# EUROPEAN PATENT APPLICATION

(11) **EP 3 854 362 A1**
(43) Date of publication of application: **28.07.2021**
(21) Application number: 19862284.7
(22) Date of filing: 18.09.2019
(51) Int. Cl.: A61F 13/475

(54) **ABSORBENT ARTICLE**

(30) Priority: 18.09.2018 JP 2018173476
(71) Applicant: DAIO PAPER CORPORATION, Shikokuchuo-shi Ehime 799-0492 (JP)
(72) Inventor: YOSHIBA Megumi, Sakura-shi, Tochigi 329-1411 (JP)
(74) Representative: Schön, Christoph
(86) International application number: PCT/JP2019/036540
(87) International publication number: WO 2020/059746

(57) **Abstract**

Problem

To suppress skin touch feeling from being deteriorated and further provide excellent wearing feeling, while ensuring sufficient adhesive strength in an adhesion part of a side sheet.

Solution

In the present invention, a pair of left and right side sheets 7 are provided over the entire length of both side parts on a skin-contact surface. A sanitary napkin 1 includes overlapped regions T where a front-surface sheet 3 and the side sheets 7 are overlapped with each other, on the both side parts of the sanitary napkin 1 along the longitudinal direction. In each of the overlapped regions T, a side-sheet embossed part 10 is formed on the side sheet 7, and an inter-sheet embossed part 9 is formed to make the side sheet 7 and the front-surface sheet 3 adhere to each other. The total area of the side sheet embossed part 10 is formed smaller than the total area of the inter-sheet embossed part 9.

## Description

### Technical field

The present invention relates to an absorbent article for use as an incontinence pad, a panty liner, a sanitary napkin and the like, and in particular relates to an absorbent article including a pair of left and right side sheets on the both side parts of the skin-contact surface side.

### Background

The absorbent article known conventionally includes an absorber made of paper cotton such as crushed pulp, and interposed between a front-surface sheet and a liquid impermeable back-surface sheet such as a polyethylene sheet or a nonwoven fabric with polyethylene sheet laminated, and further includes a pair of left and right side sheets arranged on the both side parts of the skin-contact surface side over the entire length.

The side sheets are arranged so as to have overlapped portions in which the width inside parts thereof are overlapped with the front-surface sheet in the thickness direction on the skin-contact surface side of the absorbent article, and in which adhering parts formed in various forms make the front-surface sheet and the side sheets adhere to each other. As for the adhesion, in an example, Patent Document 1 to be indicated below discloses an absorbent article including embossed parts which are formed along side sheets and which have a plurality of compressed parts. The embossed part has first embossed parts formed at the front end and rear end which are the ends in the longitudinal direction of the absorbent article, and a second embossed part formed at the center in the longitudinal direction. The first embossed part is wider than the second embossed part in the width direction orthogonal to the longitudinal direction. The compressed parts constituting the second embossed part are formed so as to be spaced apart from one another.

Patent Document 2 to be indicated below discloses an absorptive article equipped with a leakage prevention section included in a side sheet. The leakage prevention section includes a center region and a pair of end regions located further toward the outside than the center region in the longitudinal direction. The end regions include treated sections having been subjected to joining treatment which enables the portions of the side sheets which face each other to be joined together; and non-treated sections not having been subjected to the joining treatment.

Patent Document 3 to be indicated below discloses an absorbent article including compression parts that compressingly join inner ends in a width direction of respective side sheets to a surface sheet in a skin surface side of the surface sheet at positions overlapped with side areas in the width direction of an absorber, and cover sheets that cover the compression parts from the skin surface side and make the compression parts invisible from the skin surface side of the surface sheet.

### Citation List

### Patent Literature

[Patent Document 1] Japanese Patent Application Laid-Open Publication No. 2008-289535
[Patent Document 2] Japanese Patent Application Laid-Open Publication No. 2010-264161
[Patent Document 3] Japanese Patent Application Laid-Open Publication No. 2017-104436

### Summary of Invention

### Technical Problem

In the absorbent article disclosed in Patent Document 1 and the absorptive article disclosed in Patent Document 2, the joined parts for joining the side sheet and the front surface sheet are configured with the embossed parts which are compressed from the skin-contact surface side of the side sheet. Thus, it is pointed out that the embossed part having a larger area for providing higher joining strength deteriorates skin touch feeling, while the embossed part having a smaller area for suppressing the deterioration of the skin touch feeling has lower joining strength and is released easily.

In the absorbent article disclosed in Patent Document 3, the compression parts compressingly join the inner ends in the width direction of the side sheets to the surface sheet, and the cover sheets covering the compression parts are joined to the side sheets only via an adhesive layer. It is pointed out that the adhesive layer under low temperature conditions such as in winter time is easily released because the adhesive property thereof is lowered. If the adhesive layer is released, twisting and wrinkling easily occur in the laminated portion of the side sheet, and thus wearing feeling is likely to be deteriorated. It is pointed out that, in the case of using an adhesive layer as means of joining sheet members to be brought into contact with a skin surface, when the mass per unit area of an adhesive is increased in order to provide higher joining strength, the adhesive penetrates the sheet and floats on the skin side, and generates stickiness, resulting in deteriorating the wearing feeling.

The major object of the present invention is to provide an absorbent article, in which an adhesion part of a side sheet allows to suppress skin touch feeling from being deteriorated and further provide excellent wearing feeling, while ensuring sufficient adhesive strength.

### Solution to Problem

In order to solve the above problem, the invention in one aspect according to claim 1 provides an absorbent article including an absorber interposed between a front-surface sheet and a back-surface sheet, and further including a pair of left and right side sheets arranged on both side parts of a skin-contact surface side over the entire length. The front-surface sheet and the side sheets are overlapped with each other in overlapped regions on the both side parts of the absorbent article along the longitudinal direction. In each of the overlapped regions, a side-sheet embossed part is formed on the side sheet, and an inter-sheet embossed part is formed to make the side sheet and the front-surface sheet adhere to each other. A total area of the side-sheet embossed part is smaller than a total area of the inter-sheet embossed part.

In the aspect of claim 1 described above, the overlapped regions in which the front-surface sheet and the side sheets are overlapped with each other are formed on the both side parts of the absorbent article along the longitudinal direction. In each of the overlapped regions, the side-sheet embossed part is formed, which compresses only the side sheet, and the inter-sheet embossed part is formed, which makes the overlapped sheets of the side sheet and the front-surface sheet adhere to each other. In other words, the side-sheet embossed part and the inter-sheet embossed part are provided separately. As for the relation between the total areas of the respective embossed parts, the total area of the side-sheet embossed part is formed smaller than the total area of the inter-sheet embossed part. Thus, the inter-sheet embossed part formed having a relatively large area ensures sufficient adhesive strength between the side sheet and the front-surface sheet. On the other hand, the side-sheet embossed part to be brought into direct contact with the skin surface is formed to have a relatively small area, thus allowing to suppress the skin touch feeling from being deteriorated. Moreover, the side-sheet embossed part reinforces the side sheet, thus preventing the side sheet from being twisted and wrinkled, resulting in providing excellent wearing feeling.

In the aspect according to claim 2, the absorbent article of claim 1 includes the side-sheet embossed part and the inter-sheet embossed part formed in different plane patterns.

In the aspect of claim 2 described above, the formation of the side-sheet embossed part and the inter-sheet embossed part in different plane patterns allows to form, taking into consideration the functions of the respective embossed parts, the embossed parts in different plane patterns. In an example, the side-sheet embossed part is formed in the plane pattern which hardly causes deterioration of the skin touch feeling during wearing, and which provides excellent decorative appearance seen from the skin-contact surface side, while the inter-sheet embossed part is formed in the plane pattern which is designed by taking into consideration the adhesive strength between the side sheet and the front-surface sheet.

In the aspect according to claim 3, the absorbent article of claim 1 or 2 includes the inter-sheet embossed part formed in an intermittent plane pattern including compressed parts and non-compressed parts arranged alternately along the longitudinal direction of the absorbent article.

In the aspect of claim 3 described above, the formation of the inter-sheet embossed part in the intermittent plane pattern including the compressed parts and the non-compressed parts arranged alternately along the longitudinal direction of the absorbent article allows to disperse pressure at the time of compressing, and increases the adhesive strength between the side sheet and the front-surface sheet.

In the aspect according to claim 4, the absorbent article of any one of claims 1 to 3 includes the side-sheet embossed part and the inter-sheet embossed part formed so as not to be overlapped with each other in a thickness direction.

In the aspect of claim 4 described above, the formation of the side-sheet embossed part and the inter-sheet embossed part not to be overlapped with each other in the thickness direction ensures that the embossed parts respectively provide higher adhesive strength between the sheets, and that the embossed parts effectively exert their functions.

In the aspect according to claim 5, the absorbent article of any one of claims 1 to 4 includes the side-sheet embossed part formed to have a cross-sectional shape bulging out toward a skin side.

In the aspect of claim 5 described above, the formation of the side-sheet embossed part in the cross-sectional shape bulging out toward the skin side provides soft skin touch feeling.

In the aspect according to claim 6, the absorbent article of any one of claims 1 to 5 includes the inter-sheet embossed part formed as a recessed part by recessing toward the skin side in a non-skin-contact surface side of the front-surface sheet.

In the aspect of claim 6 described above, the formation of the inter-sheet embossed part as the recessed part by recessing toward the skin side in the non-skin-contact surface side of the front-surface sheet hardly allows the inter-sheet embossed part to be brought into contact with the skin surface, thus suppressing the skin touch feeling from being deteriorated, resulting in providing excellent wearing feeling.

In the aspect according to claim 7, the absorbent article of any one of claims 1 to 6 includes the side-sheet embossed part formed to have a cross-sectional shape bulging out toward the skin side continuously along the longitudinal direction of the absorbent article, and further have an elastic stretchable member inside a space bulging out toward the skin side.

In the aspect of claim 7 described above, the arrangement of the elastic stretchable member in the recessed part of the side-sheet embossed part allows the elastic stretchable member to easily exhibit the shrinkage force thereof, allows the side sheet to easily stand toward the skin side, and further allows the absorbent article to curve in the longitudinal direction.

In the aspect according to claim 8, the absorbent article of any one of claims 1 to 7 includes the side sheet formed in one layer or a plurality of layers in the overlapped region.

In the aspect of claim 8 described above, in the overlapped region of the front-surface sheet and the side sheet formed on the both side parts of the absorbent article, the side sheet is configured in one layer or in two or more layers.

### Advantageous Effect of Invention

As detailed above, the present invention allows to suppress the skin touch feeling from being deteriorated and further provide excellent wearing feeling, while ensuring sufficient adhesive strength, in the adhesion part of the side sheet.

### Brief Description of Drawings

Fig. 1 is a partially broken developed view of a sanitary napkin 1 according to the present invention.
Fig. 2 is a diagram taken along a II-II line of Fig. 1.
Fig. 3 is an enlarged plan view of a laminated portion 8 of a side sheet 7.
Fig. 4 is a diagram taken along a IV-IV line of Fig. 3.
Fig. 5 shows plan views of an inter-sheet embossed part 9.
Fig. 6 shows plan views of modifications of a compressed part 9a.
Fig. 7 is a plan view of a modification of a side-sheet embossed part 10.
Fig. 8 shows plan views of the side-sheet embossed part 10 in modifications.
Fig. 9 is a plan view of the sanitary napkin 1 in a modification.
Fig. 10 is a diagram taken along an X-X line of Fig. 9.
Fig. 11 is a side view illustrating a machine for processing an embossed part.
Fig. 12 is a cross-sectional view of an overlapped region T in which a front-surface sheet 3 and the side sheet 7 are overlapped with each other in another embodiment.

### Description of Embodiments

Hereinafter, some embodiments according to the present invention will be detailed with reference to the drawings.

### One example of basic configuration of sanitary napkin

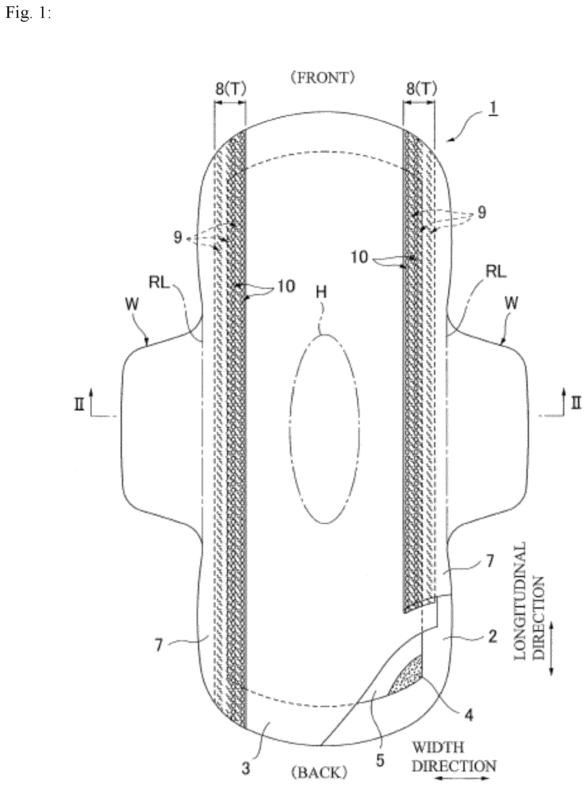

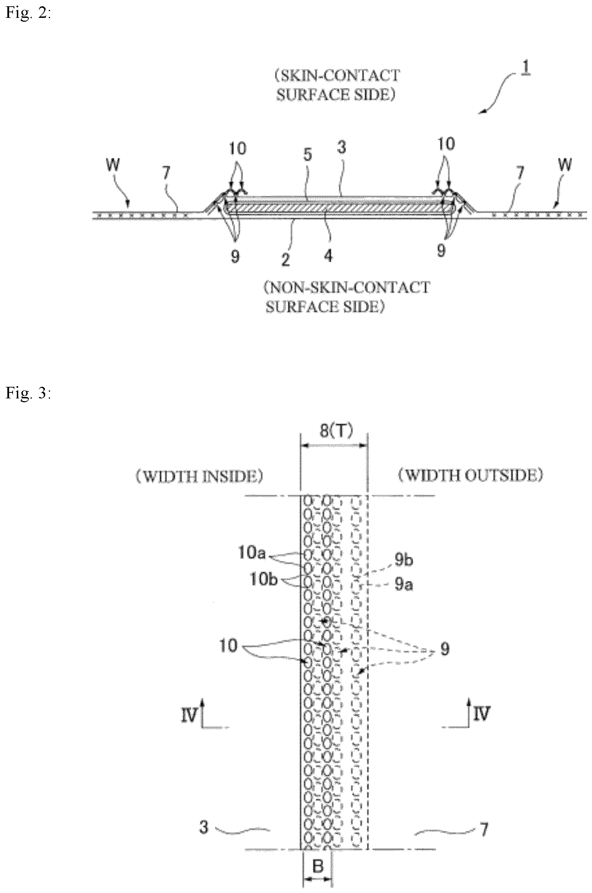

As shown in Fig. 1 and Fig. 2, the sanitary napkin 1 according to the present invention mainly includes a liquid impermeable back-surface sheet 2 made of a polyethylene sheet or the like, the front-surface sheet 3 which serves as a skin-contact surface and allows body fluid to promptly pass through, an absorber 4 which is interposed between the both sheets 2, 3 and is made from cotton-like pulp, synthetic pulp or the like, an encapsulating sheet 5 which is made of a crepe paper sheet, nonwoven fabric or the like and covers at least the skin-side surface and the non-skin-side surface of the absorber 4 in order to retain the shape of the absorber 4 and improve the diffusibility thereof, and the left and right side sheets 7 in a pair which are arranged respectively on the both side parts of the skin-contact side along the longitudinal direction thereof over the substantially entire length of the sanitary napkin 1. Around the absorber 4, along the upper and lower end edge parts of the absorber 4, the outer edge parts of the back-surface sheet 2 and the front-surface sheet 3 are made to adhere to each other by adhesion means such as an adhesive such as hot melt, heat seal, or ultrasonic sealing. Along the both side edge parts thereof, the parts extending laterally over the absorber 4 of the back-surface sheet 2 and the side sheets 7 are made to adhere to each other by adhesion means such as an adhesive such as hot melt, heat seal, or ultrasonic sealing. Although, in the illustrated example, the absorber 4 is configured in one layer, the absorber 4 may be configured in a plurality of layers to have a middle-height portion, or may be configured in a plurality of layers in which absorbers in identical size and shape are laminated. A hydrophilic second sheet may be interposed between the front-surface sheet 3 and the absorber 4 when necessary.

The back-surface sheet 2 is made from sheet material, such as polyethylene, having at least water shielding property. Recently, material having moisture permeability tends to be used from the viewpoint of stuffiness prevention. As such water shielding and moisture permeable sheet material, a microporous sheet is suitably used, which is obtained by melt-kneading inorganic filler in olefin resin such as polyethylene or polypropylene and forming a sheet, and thereafter extending the sheet in one axial direction or two axial directions. The back-surface sheet 2 includes an adhesive layer (not shown) in one line or a plurality of lines on the non-skin-contact surface side (outer surface) thereof, to allow the sanitary napkin 1 to be fixed on a bottom underwear during wearing on the body. The back-surface sheet 2 described above may be made from poly-laminated nonwoven fabric in which a plastic film and nonwoven fabric are laminated.

The front-surface sheet 3 described above is suitably made from porous or nonporous nonwoven fabric, a porous plastic sheet or the like. Examples of the material fiber included in the nonwoven fabric are not only synthetic fiber such as olefinic fiber such as polyethylene and polypropylene, polyester, and polyamide, but also recycled fiber such as rayon and cupra, and natural fiber such as cotton. The nonwoven fabric for use is made by appropriate processing method, such as spunlacing method, spunlaying method, thermal bonding method, meltblowing method, or needlepunching method. In these processing methods, the spunlacing method allows to make nonwoven fabric excellent in flexibility and drape, and the thermal bonding method allows to make bulky nonwoven fabric excellent in compression restorability. In the case of the front-surface sheet 3 having a large number of through holes, the front-surface sheet 3 allows body fluid to be promptly absorbed, and thus provides excellent dry touch. The fiber of the nonwoven fabric may be either long or short, but is preferably short in order to produce towel cloth feeling. In order to facilitate embossing, olefinic fiber can be suitably used, such as polyethylene or polypropylene having a relatively low melting point. Alternatively, composite fiber is preferably used, of sheath/core fiber with high-melting point fiber as a core and low-melting point fiber as a sheath, side-by-side fiber, and splittable fiber.

The absorber 4 is capable of absorbing and holding body fluid. The absorber 4 may include granular powder superabsorbent polymer dispersed in fluffed pulp fiber, or may be made of a polymer sheet with superabsorbent polymer supported between the top layer sheet of the skin-contact surface side and the lower layer sheet of the non-skin-contact surface side.

Examples of the pulp fiber are cellulose fiber such as chemical pulp and melted pulp made from wood, and artificial cellulose fiber such as rayon and acetate. Needle leaved tree pulp having longer fiber than that of broad leaved tree pulp is suitably used from the viewpoint of function and cost. The pulp fiber may preferably have a mass per unit area in the range from 150 g/m² to 700 g/m², preferably in the range from 250 g/m² to 400 g/m². The superabsorbent polymer may preferably have a mass per unit area in the range from 15 g/m² to 470 g/m², preferably in the range from 15 g/m² to 100 g/m².

Examples of the superabsorbent polymer are crosslinking polyacrylate, self-crosslinking polyacrylate, saponified substance of crosslinking copolymer of acrylic acid ester and vinyl acetate, crosslinking substance of copolymer of isobutylene and maleic anhydride, crosslinking polysulfonate, and partially crosslinking substance of water swellable polymer such as polyethylene oxide and polyacrylamide. Among them, substance of acryl acid or acrylate-based substance is suitably used, which are excellent in water absorption amount and water absorption rate. The absorption ratio (water absorbing power) and the absorption rate of the superabsorbent polymer having water absorbing property are adjustable, by adjusting the cross-linking density and the cross-linking density gradient in its manufacturing process.

### Side Sheet

In the illustrated example, the front-surface sheet 3 is formed slightly wider than the absorber 4, just enough to cover the absorber 4. The outer sides of the front-surface sheet 3 in the width direction are covered by the side sheets 7 (different members from the front-surface sheet 3) respectively extending from the both side parts of the skin-contact surface of the front-surface sheet 3. The side sheets 7 may be made from the nonwoven material subjected to appropriate water repellent treatment or hydrophilic treatment depending on the purpose of preventing penetration of body fluid or improving skin touch feeling. Although the side sheets 7 may be formed from natural fiber, synthetic fiber, recycled fiber or the like by an appropriate processing method, the side sheets 7 may be preferably formed from the nonwoven fabric having a small mass per unit area and having air permeability so as to provide good touch feeling free from rough feeling and prevent stuffiness. Specifically, the side sheets 7 may be preferably formed from the nonwoven fabric having a mass per unit area in the range of 8 g/m² to 23 g/m².

As shown in Fig. 2, the side sheets 7 are arranged in the regions outside the central portion in the width direction of the sanitary napkin 1, from the predetermined inside positions overlapped with the absorber 4 in the thickness direction to the outer edge parts of the back-surface sheet 2 beyond the side edge parts of the absorber. The laminated sheet portions of the side sheets 7 and the back-surface sheet 2 are formed as flap portions free from the absorber 4, in the both sides of the absorber 4. As shown in Fig. 1, the flap portions are formed as a pair of left and right wing-shaped flaps W, W at the side positions of the region substantially corresponding to a body fluid expelled portion H of a wearer. Each of the wing-shaped flaps W, W has an adhesive layer (not shown) on the outer surface side thereof. When attached to a panty, the wing-shaped flaps W, W are folded to the non-skin-contact side along folding lines RL of the base edges thereof, and adheres so as to wrap the crotch portion of the panty.

Each of the width inside parts of the side sheets 7 forms a laminated portion 8 laminated in a plurality of layers. The laminated portions 8 of the side sheets 7 may be formed by folding the width inside parts of the side sheets 7 in the width direction, or by laminating a plurality of sheets into layers. Each of the laminated portions 8 is formed along the longitudinal direction of the sanitary napkin 1 with a substantially equal width over the entire length of the side sheet 7, in the range in the width direction including the region (overlapped region T) in which the front-surface sheet 3 and the side sheet 7 are overlapped with each other in the thickness direction. That is, the width inside parts of the laminated portions 8 have the overlapped portions overlapped with the front-surface sheet 3 in the thickness direction. Preferably, the entire laminated portions 8 are overlapped with the front-surface sheet 3 in the thickness direction. More preferably, as shown in the illustrated example, the laminated portions 8 of the side sheets 7 substantially correspond to the overlapped regions T of the front-surface sheet 3 and the side sheets 7.

### Embossed Part

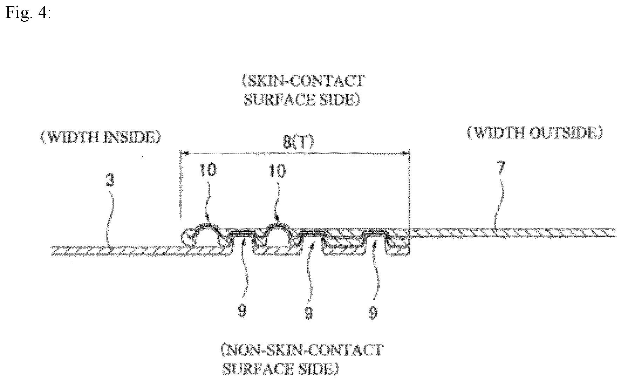

As shown in Fig. 3 and Fig. 4, each of the laminated portions 8 (overlapped regions T) of the side sheets 7 has a side-sheet embossed part 10 in which the layers of the side sheet 7 corresponding to the laminated portion 8 are made to adhere to each other, and an inter-sheet embossed part 9 in which the side sheet 7 corresponding to the laminated portion 8 and the front-surface sheet 3 are made to adhere to each other. The side-sheet embossed part 10 corresponds to the embossed part in which only the layers of the side sheet 7 are integrated so as to prevent the side sheet 7 from being twisted and wrinkled in the laminated portion 8, and further so as to provide excellent decorative appearance when seen from the skin-contact surface side of the sanitary napkin 1. The inter-sheet embossed part 9 corresponds to the structurally-embossed part which makes the side sheet 7 and the front-surface sheet 3 firmly adhere to each other.

The inter-sheet embossed part 9 and the side-sheet embossed part 10 are formed in the manner that fiber is thermally fused by applying heat or ultrasonic wave in compressing. From the viewpoint of producing soft skin touch, ultrasonic wave may be preferably used as thermally fusing means at least for the side-sheet embossed part 10.

The inter-sheet embossed part 9 and the side-sheet embossed part 10 are formed so that the total area of the side-sheet embossed part 10 is smaller than the total area of the inter-sheet embossed part 9. Each of the total areas is obtained by summing the areas of the bottoms of the recessed parts of the inter-sheet embossed part 9 or the side-sheet embossed part 10 with respect to the whole area of the sanitary napkin 1. As for a total area A₁₀ of the side-sheet embossed part 10 and a total area A₉ of the inter-sheet embossed part 9, the ratio A₁₀/A₉ between them may be preferably 1/10 to 9/10, preferably 3/10 to 6/10.

As described above, on the sanitary napkin 1, the total area of the side-sheet embossed part 10 is smaller than the total area of the inter-sheet embossed part 9. Thus, the inter-sheet embossed part 9 formed to have a relatively large area ensures sufficient adhesive strength between the side sheet 7 and the front-surface sheet 3. On the other hand, the side-sheet embossed part 10 which is brought into direct contact with the skin surface is formed to have a relatively small area, thereby allowing to suppress the skin touch feeling from being deteriorated, and further allowing to prevent the laminated portion 8 of the side sheet 7 from being twisted and wrinkled, resulting in providing excellent wearing feeling.

Further, since the side-sheet embossed part 10 is visible from the skin-contact surface side, the appearance of the sanitary napkin 1 when seen from the skin-contact surface side is improved, resulting in providing excellent decorative appearance. In particular, the side-sheet embossed part 10 is formed preferably in a wavy plane pattern or formed preferably with an additional pattern such as flowers, so as to provide excellent decorative appearance.

As shown in Fig. 3, the inter-sheet embossed part 9 and the side-sheet embossed part 10 may be preferably formed in different plane patterns. This allows to the formation of the embossed parts 9, 10 in different plane patterns designed by taking into consideration respective functions. That is, the inter-sheet embossed part 9 may be preferably formed in the plane pattern allowing to make the side sheet 7 and the front-surface sheet 3 firmly adhere to each other, and the side-sheet embossed part 10 may be preferably formed in the plane pattern allowing to suppresses the skin touch feeling from being deteriorated and to provide excellent decorative appearance.

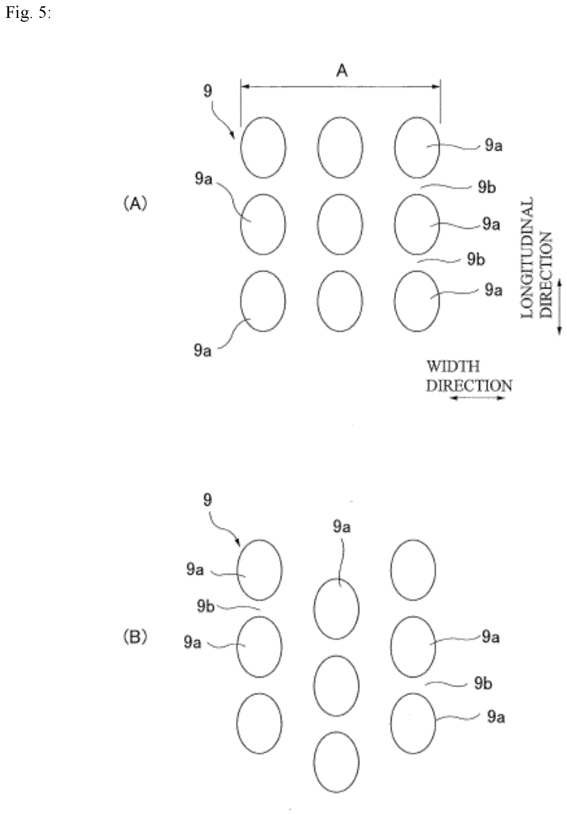

As shown in Fig. 5, the inter-sheet embossed part 9 may be preferably formed along the longitudinal direction of the sanitary napkin 1, in the intermittent plane pattern in which compressed parts 9a and non-compressed parts 9b are arranged alternately. The formation of the inter-sheet embossed part 9 in the intermittent pattern disperses the pressure at the time of compressing the inter-sheet embossed part 9, thus enabling to increase the adhesive strength between the side sheet 7 and the front-surface sheet 3. Even under the state where the side sheet 7 and the front-surface sheet 3 are made to firmly adhere to each other by the inter-sheet embossed part 9, the non-compressed parts 9b allow the sanitary napkin 1 to be easily curved, and thus the sanitary napkin 1 is flexibly deformed and easily fits the skin surface. The inter-sheet embossed part 9 may be formed in a continuous line continuing in the longitudinal direction of the sanitary napkin 1.

As shown in Fig. 5, the inter-sheet embossed part 9 formed along the longitudinal direction may be arranged in one line or a plurality of lines in the width direction of the sanitary napkin 1. In the illustrated example, three lines are arranged in the width direction. The arrangement of the inter-sheet embossed part 9 in a plurality of lines in the width direction as in the illustrated example allows to increase the adhesive strength between the side sheet 7 and the front-surface sheet 3. As for the arrangement of the plurality of compressed parts 9a in the width direction, the compressed parts 9a may be arranged in the square lattice shape, as shown in Fig. 5A, in which adjacent compressed parts 9a are aligned along the longitudinal direction and the width direction, or may be arranged in the rhombic lattice shape, as shown in Fig. 5B, in which compressed parts 9a adjacent in the width direction are aligned so as to be shifted by a half pitch in the longitudinal direction. The arrangement in the square lattice shape allows the laminated portion 8 to be easily curved along a round shape of the front-back body. The arrangement in the rhombic lattice shape allows the laminated portion 8 to be easily deformed in the oblique direction.

As shown in Fig. 5, the inter-sheet embossed part 9 has, as an applied width A, an arbitrary width within the width of the laminated portion 8, preferably a width in the range of 3 mm to 15 mm, more preferably a width in the range of 5 mm to 10 mm.

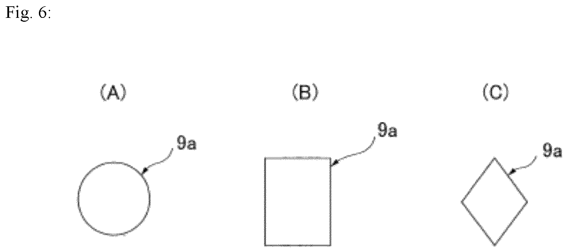

In the examples shown in Fig. 5, the plane shape of the compressed parts 9a of the inter-sheet embossed part 9 is an oval shape long in the longitudinal direction of the sanitary napkin1. Alternatively, the shape may be, as shown in Fig. 6, A: a round shape, B: a rectangular shape, or C: a diamond shape.

The inter-sheet embossed part 9 may be formed by compressing the front-surface sheet 3 and the side sheet 7 in the state of being laminated to each other, from the outer surface side of the front-surface sheet 3 or the outer surface side of the side sheet 7. That is, the sheets may be compressed from either direction, and may be compressed so as to be swollen to the opposite side of compressing, or so as not to be swollen.

In a preferred embodiment, as shown in Fig. 4, the inter-sheet embossed part 9 is formed as a recessed part, by recessing toward the skin side in the non-skin-contact surface side of the front-surface sheet 3 with a compression depth smaller than the laminated thickness of the laminated portion 8 of the side sheet 7 and the front-surface sheet 3. That is, the inter-sheet embossed part 9 may be preferably formed by making the laminated body of the side sheet 7 and the front-surface sheet 3 pass through between the embossing roll which is arranged in the side facing the front-surface sheet 3 and has convex parts corresponding to the inter-sheet embossed part 9, and the embossing roll which is arranged in the side facing the side sheet 7 and has flat parts facing to the convex parts. This forms the recessed parts which are recessed in the thickness direction in the non-skin-contact surface side of the front-surface sheet 3, and forms substantially flat parts on the skin-contact surface side of the side sheet 7. Accordingly, the inter-sheet embossed part 9 is formed to have the cross-sectional shape free from any protrusion protruding toward the skin side above the skin-contact surface of the side sheet 7, and thus the inter-sheet embossed part 9 is hardly brought into contact with the skin surface, resulting in suppressing the skin touch feeling from being deteriorated and providing excellent wearing feeling.

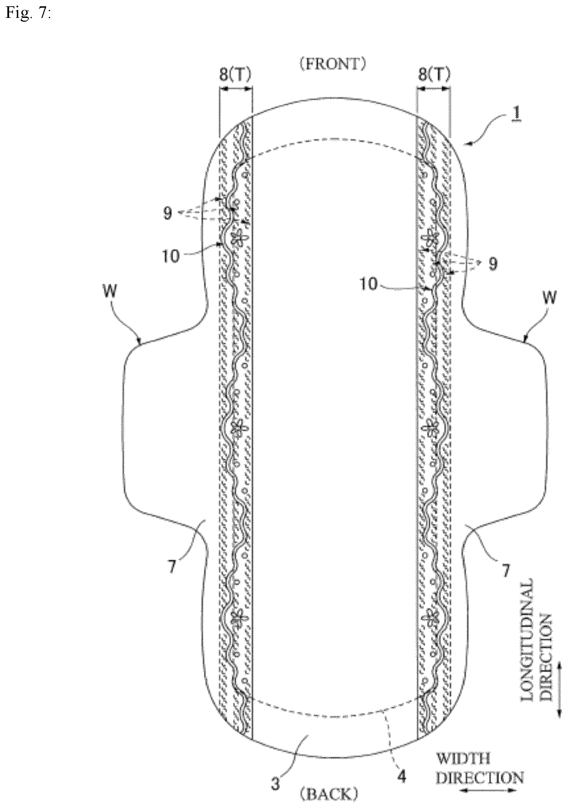

The side-sheet embossed part 10 is described next. The plane pattern of the side-sheet embossed part 10 may be formed in the intermittent pattern as shown in Fig. 1 and Fig. 3, in which compressed parts 10a and non-compressed parts 10b are alternately arranged substantially along the longitudinal direction of the sanitary napkin 1, or alternatively in the continuous pattern as shown in Fig. 7, in which compressed parts are continuously arranged.

In the embodiment shown in Fig. 1 and Fig. 3, the side-sheet embossed part 10 is formed in the intermittent pattern, and the plane shape of the compressed parts 10a is an oval shape long in the longitudinal direction of the sanitary napkin 1, as in the case of the inter-sheet embossed part 9. The plane shape of the compressed parts 10a may be a round shape, a rectangular shape, or a diamond shape (refer to Fig. 6). The side-sheet embossed part 10 formed in the intermittent pattern having the compressed parts 10a and the non-compressed parts 10b alternately arranged along the longitudinal direction may be arranged in one line or a plurality of lines spaced apart from one another in the width direction. In the embodiment shown in Fig. 3, the side-sheet embossed part 10 is arranged in two lines spaced apart from each other in the width direction.

As shown in Fig. 7, the side-sheet embossed part 10 may be formed in a continuous pattern substantially along the longitudinal direction of the sanitary napkin 1. In the case of the side-sheet embossed part 10 formed in such a continuous pattern, the side-sheet embossed part 10 may be formed in one line as shown in the illustrated example, or may be formed in a plurality of lines, not shown, spaced apart from one another in the width direction.

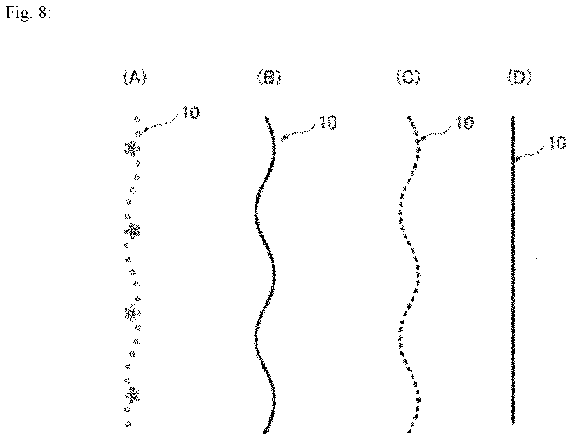

As shown in Fig. 7 and Fig. 8A to Fig. 8C, the side-sheet embossed part 10 may preferably include a wavy line waving in the width direction substantially along the longitudinal direction of the sanitary napkin 1. The side-sheet embossed part 10 including such a wavy line hardly hinders the laminated portion 8 from being deformed along the body shape during wearing, and provides excellent decorative appearance. To provide further excellent decorative appearance, as shown in Fig. 7 and Fig. 8A, the side-sheet embossed part 10 may be formed with compressed parts having concrete shapes such as flowers, stars or heart shapes, or geometrical shapes such as circles or polygons discretely arranged on the wavy line or in the vicinity of the wavy line. The wavy side-sheet embossed part 10 may be formed in a continuous line as shown in Fig. 7 and Fig. 8B, or may be formed in an intermittent line as shown in Fig.8A and Fig. 8C.

As shown in Fig. 8D, the side-sheet embossed part 10 may be formed in a straight line of a continuous line or an intermittent line along the longitudinal direction of the sanitary napkin 1.

As shown in Fig. 3, an applied width B of the side-sheet embossed part 10 may be preferably smaller than the applied width A of the inter-sheet embossed part 9, specifically in the range of 1 mm to 10 mm, preferably in the range of 1 mm to 5 mm.

As shown in Fig. 3, the inter-sheet embossed part 9 and the side-sheet embossed part 10 may be preferably formed so as not to be overlapped with each other in the thickness direction. This ensures that the embossed parts 9, 10 respectively provide higher adhesive strength between the sheets, and that the embossed parts 9, 10 effectively exert their functions. In the embodiment shown in Fig. 3, two lines of the side-sheet embossed part 10 are arranged so as to be spaced apart from each other in the width direction in the inside of the laminated portion 8, and one line of the inter-sheet embossed part 9 is arranged between the two lines of the side-sheet embossed part 10, and further two lines of the inter-sheet embossed part 9 are arranged outside the side-sheet embossed part 10 in the width direction. As described above, the inter-sheet embossed part 9 may be preferably formed in lines spaced apart from one another by substantially equal intervals over substantially the entire width in the width direction of the laminated portion 8, and the side-sheet embossed part 10 may be preferably formed in the width inside part from the width center of the laminated portion 8. This allows the inter-sheet embossed part 9 to make the side sheet 7 and the front-surface sheet 3 adhere to each other in a wider range, and allows the side-sheet embossed part 10 to make the portions of the side sheet 7 where twisting and wrinkling likely occurs, to adhere to each other. As shown in Fig. 7, in the case of the side-sheet embossed part 10 formed in a wavy plane pattern, it is preferable that the inter-sheet embossed part 9 may not be arranged at the positions where the side-sheet embossed part 10 is arranged.

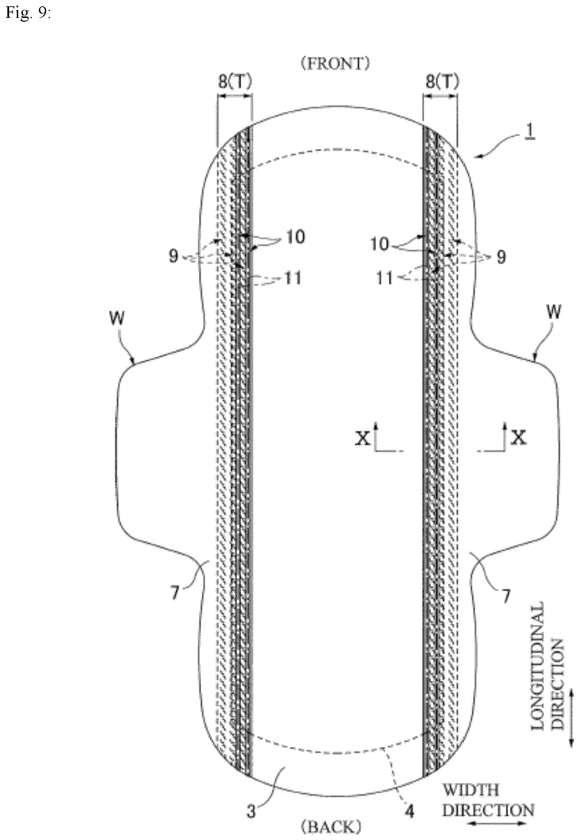

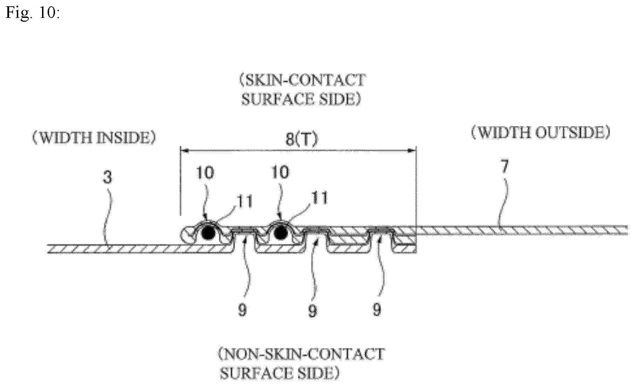

As shown in Fig. 9 and Fig. 10, the side-sheet embossed part 10 may be formed to have the cross-sectional shape bulging out toward the skin side continuously along the longitudinal direction of the sanitary napkin 1, and may have an elastic stretchable member 11 arranged in a stretched state inside the space bulging out toward the skin side. This allows the elastic stretchable member 11 to exhibit the shrinkage force thereof. Thus, the width inside part of the side sheet 7 with the elastic stretchable member11 arranged therein stands toward the skin side, thus easily serving as a barrier which prevents the body fluid from flowing sideways on the surface of the front-surface sheet 3. Further, the sanitary napkin 1 easily curves in the longitudinal direction. The elastic stretchable member 11, which is rubber thread preferably, is made to adhere along the top portion of the inside of the side-sheet embossed part 10 which bulges out toward the skin side in a dome-like sectional shape, by an adhesive at the both ends or at appropriate intervals in the longitudinal direction.

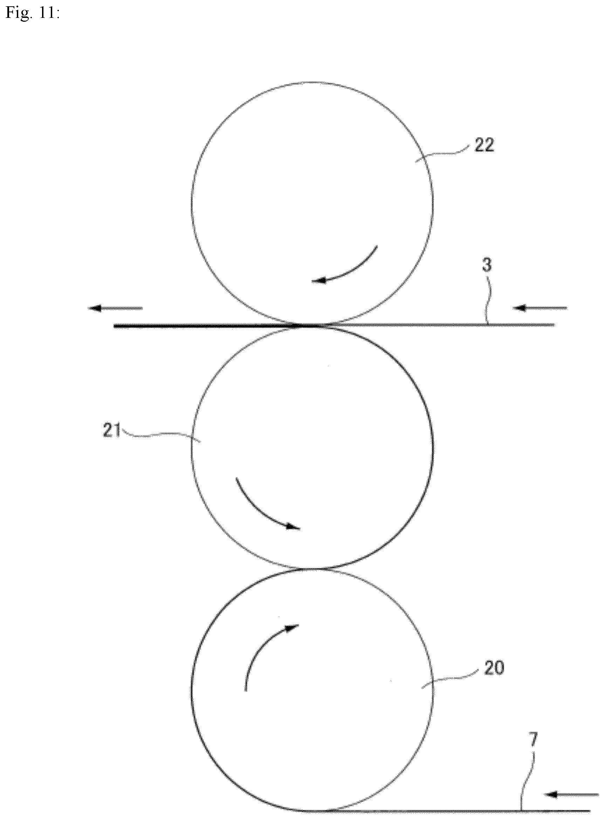

In the manufacturing method of the sanitary napkin 1, an adhesion method between the front-surface sheet 3 and the side sheet 7 is described next. As shown in Fig. 11, the width inside part of the side sheet 7 is folded and laminated into plural layers to form the laminated portion 8 of the side sheet, and thereafter the side-sheet embossed part 10 is formed on the laminated portion 8 of the side sheet. The side-sheet embossed part 10 is formed by making the laminated portion 8 of the side sheet 7 pass through between a first roll 20 which has a large number of convex parts corresponding to the side-sheet embossed part 10 on its peripheral surface, and a second roll 21 which has a large number of recessed parts corresponding to the convex parts on its peripheral surface. Thereafter, the front-surface sheet 3 is laminated on the side sheet 7 which is in the state of being wrapped around the second roll 21, and the laminated sheets are made to pass through between the second roll 21 and a third roll 22 which has a large number of convex parts corresponding to the inter-sheet embossed part 9 on its peripheral surface. This allows to compress the front-sheet surface 3 and the laminated portion 8 of the side sheet 7 between the convex parts of the third roll 22 and the flat portion of the second roll 21, to form the inter-sheet embossed part 9.

In the embodiment described above, the side-sheet embossed part 10 is formed by making the laminated portion 8 of the side sheet 7 pass through between the first roll 20 having a large number of convex parts and the second roll 21 having a large number of recessed parts, so that the laminated portion 8 of the side sheet 7 bulges toward the skin side. Alternatively, the side-sheet embossed part 10 may be formed so as to be recessed by a depth smaller than the thickness of the laminated portion 8 of the side sheet 7, by making the laminated portion 8 pass through between the first roll 20 having a large number of convex parts and the second roll 21 having the flat parts corresponding to the convex parts, to compress the laminated portion 8 from the skin-contact surface side or the non-skin-contact surface side of the side sheet 7. In the embodiment described above, the inter-sheet embossed part 9 is formed so as to be recessed by a depth smaller than the thickness of the front-surface sheet 3 and the laminated portion 8 of the side sheet 7, by making the front-surface sheet 3 and the side sheet 7 pass through between the third roll 22 having a large number of convex parts and the second roll 21 having the flat parts corresponding to the convex parts, to compress the sheets from the non-skin-contact surface side of the front-surface sheet 3. Alternatively, the inter-sheet embossed part 9 may be formed so that the front-surface sheet 3 and the laminated portion 8 of the side sheet 7 integrally bulge toward the skin side or the non-skin side, by making the sheets pass through between the roll having convex parts and the roll having recessed parts corresponding to the convex parts.

### Other embodiments

The laminated portion 8 of the side sheet 7 in the embodiment described above is formed in two layers, or may be formed in three or more layers. In the case of the laminated portion 8 in three or more layers, the inter-sheet embossed part 9 may make at least the lowest layer of the side sheet 7 and the front-surface sheet 3 adhere to each other, or may make the layers of the side sheet 7 and the front-surface sheet 3 integrally adhere to one another. The formation of the laminated portion 8 in three or more layers increases the thickness of the laminated portion 8, and thus increases the effect as barriers along the both side parts of the front-surface sheet 3.

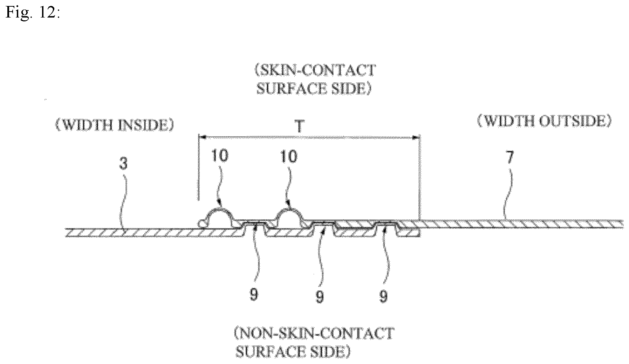

In the embodiment described above, in the overlapped region T which is formed in the both side parts of the sanitary napkin 1 and in which the front-surface sheet 3 and the side sheet 7 are overlapped with each other, the laminated portion 8 is formed by folding the side sheet 7 into layers. Alternatively, as shown in Fig. 12, the side sheet 7 is formed just in one layer. In this case, the side-sheet embossed part 10 is formed in the overlapped region T only on the side sheet 7 in one layer. The formation of the side-sheet embossed part 10 reinforces the side sheet 7 in the overlapped region T, thus suppressing the side sheet 7 from being twisted and wrinkled in the region, resulting in providing excellent wearing feeling.

1: SANITARY NAPKIN, 2: BACK-SURFACE SHEET, 3: FRONT-SURFACE SHEET, 4: ABSORBER, 5: ENCAPSULATING SHEET, 7: SIDE SHEET, 8: LAMINATED PORTION, 9: INTER-SHEET EMBOSSED PART, 10: SIDE-SHEET EMBOSSED PART, 11: ELASTIC STRETCHABLE MEMBER

## Claims

1. An absorbent article comprising an absorber interposed between a front-surface sheet and a back-surface sheet, the absorbent article further comprising a pair of left and right side sheets arranged on both side parts of a skin-contact surface side over an entire length, wherein
the front-surface sheet and the side sheets are overlapped with each other in overlapped regions on the both side parts of the absorbent article along a longitudinal direction,
in each of the overlapped regions, a side-sheet embossed part is formed on the side sheet, and an inter-sheet embossed part is formed to make the side sheet and the front-surface sheet adhere to each other, and wherein
a total area of the side-sheet embossed part is smaller than a total area of the inter-sheet embossed part.

2. The absorbent article according to claim 1, wherein
the side-sheet embossed part and the inter-sheet embossed part are formed in different plane patterns.

3. The absorbent article according to claim 1 or 2, wherein
the inter-sheet embossed part is formed in an intermittent plane pattern including compressed parts and non-compressed parts arranged alternately along the longitudinal direction of the absorbent article.

4. The absorbent article according to any one of claims 1 to 3, wherein
the side-sheet embossed part and the inter-sheet embossed part are formed so as not to be overlapped with each other in a thickness direction.

5. The absorbent article according to any one of claims 1 to 4, wherein
the side-sheet embossed part is formed to have a cross-sectional shape bulging out toward a skin side.

6. The absorbent article according to any one of claims 1 to 5, wherein
the inter-sheet embossed part is formed as a recessed part by recessing toward the skin side in a non-skin-contact surface side of the front-surface sheet.

7. The absorbent article according to any one of claims 1 to 6, wherein
the side-sheet embossed part is formed to have a cross-sectional shape bulging out toward the skin side continuously along the longitudinal direction of the absorbent article, and has an elastic stretchable member inside a space bulging out toward the skin side.

8. The absorbent article according to any one of claims 1 to 7, wherein
in the overlapped region, the side sheet is formed in one layer or a plurality of layers.
